# NEUE EUROPÄISCHE PATENTSCHRIFT

(11) **EP 0 571 771 B2**
(45) Veröffentlichungstag und Bekanntmachung des Hinweises auf die Entscheidung über den Einspruch: **20.10.2004**
(45) Hinweis auf die Patenterteilung: 12.07.2000
(21) Anmeldenummer: 93106848.0
(22) Anmeldetag: 28.04.1993
(51) Int. Cl.: C12N 7/04, A61L 2/00

(54) **Verfahren zur Inaktivierung von Viren in Präparationen von Proteinen**
Method of inactivation of viruses in proteinic preparations
Méthode d'inactivation de virus dans des préparations protéiniques

(30) Priorität: 26.05.1992 DE 4217355; 28.11.1992 DE 4240103
(43) Veröffentlichungstag der Anmeldung: 01.12.1993
(73) Patentinhaber: Dade Behring Marburg GmbH, 35001 Marburg (DE)
(72) Erfinder: Keuper, Hermann, W-3552 Wetter (DE); Matzmorr, Walter, W-3556 Niederweimar (DE); Freudenberg, Wilfried, W-3553 Cölbe-Schönstadt (DE)

(56) Entgegenhaltungen:
- EP-A- 0 124 506
- EP-A- 0 198 299
- EP-A- 0 278 776
- EP-A- 0 384 305
- WO-A-92/00767
- VOX SANGUINIS, Bd.62, Nr.1, Februar 1992, BASEL CH Seiten 12 - 20 CHARM S E; LANDAU S; WILLIAMS B; HOROWITZ B; PRINCE A M; PASCUAL D ' HIGH-TEMPERATURE SHORT-TIME HEAT INACTIVATION OF HIV AND OTHER VIRUSES IN'
- BIOTECHNOLOGY ABSTRACTS,London,GB DBA Accession No.: 88-04264 Charm S E; Landau S H 'Heat destruction of...'

## Beschreibung

Die Erfindung betrifft ein Verfahren zur Inaktivierung von Viren in Präparationen von Proteinen. Dazu wird eine Lösung der Präparation eines Proteins kurzzeitig erhitzt.

Proteine im Sinne der Erfindung sind Plazenta-Proteine. Ein solches Protein ist beispielsweise Gewebe-Thromboplastin.

Zur Bestimmung der Thromboplastinzeit nach Quick werden Reagenzien eingesetzt, die Gewebe-Thromboplastin als aktiven Bestandteil enthalten. Der Quickwert ist ein wichtiger Screening-Test in der Gerinnungsdiagnostik.

Außerdem wird der therapeutische Einsatz von GewebeThromboplastin als "FVIII-bypassing agent" zur Behandlung der Hemmkörper-Hämophilie diskutiert. Für diese Anwendung ist die Virussicherheit der Präparation eine unabdingbare Voraussetzung. Aber auch beim Einsatz von Gewebe-Thromboplastin als Diagnostikum wird Virussicherheit im Hinblick auf die Gesundheitsvorsorge für den Anwender gefordert.

Gegenwärtig werden Gewebe-Thromboplastine zur Herstellung von Quick-Reagenzien üblicherweise aus Hirn oder Plazenta von Säugetieren gewonnen. Kontaminationen durch Viren wie HBV, HCV, HIV für Präparate humanen Ursprungs oder des Erregers von BSE im Fall des Rindes sind in derartigen Präparationen grundsätzlich nicht auszuschließen. Dadurch gewinnt ein Verfahren zur Virusinaktivierung in Gewebe-Thromboplastin-Präparaten große Bedeutung.

Bisherige Versuche zur Virusinaktivierung durch etablierte Verfahren (Detergentien, Hypochlorit, UV/gamma-Bestrahlung etc.) scheiterten an der großen Empfindlichkeit der Präparationen. Insbesondere waren alle Versuche zur Pasteurisierung oder Trockenerhitzung erfolglos.

Überraschend stellte sich heraus, daß eine Kurzzeiterhitzung beispielsweise in einem Gerät nach Chem.-Ing.-Tech. 62 (1990), 486-487 (deutsche Patentanmeldung 39 05 066) die Eigenschaften von Gewebe-Thromboplastin nicht nachteilig beeinflußt, Viren dagegen vollständig inaktiviert.

US 4,975,246 beschreibt ein Verfahren zur Inaktivierung von Mikroorganismen unter der Verwendung von Mikrowellen, um möglicht Kurze Erhitzungszeiten zu erreichen.

In den bekannten Verfahren zur Virusinaktivierung (Pasteurisierung, "Trockenerhitzung") wird üblicherweise mindestens eine Minute, meist aber mehrere Stunden erhitzt.

Gegenstand der Erfindung ist ein Verfahren zur Inaktivierung von Viren in einer Präparation eines Proteins, dadurch gekennzeichnet, daß eine Lösung dieser Präparation kurzzeitig erhitzt wird.

Es wird in einem Wärmeübertrager mit indirekter Beheizung erhitzt. Geeignet sind Wärmeübertrager beliebiger Bauformen, wie Plattenübertrager oder Rohrbündelübertrager.

Besonders geeignet ist ein Wärmeübertrager nach der deutschen Patentanmeldung 39 05 066, weil bei einem großen Wärmedurchgangskoeffizienten eine kurze Verweilzeit und eine niedere Wandtemperatur möglich ist.

Dieser Wärmeübertrager ist ein Wärmeaustauschermodul aus gestapelten Metallfolien mit dazwischen angeordneten Abstandshaltern, wobei die Metallfolien aus Metallkarten bestehen, die an gegenüberliegenden Seiten mit jeweils mindestens 2 Öffnungen versehen sind, die Abstandshalter aus Gewebekarten mit Öffnungen bestehen, die mit den Metallkarten deckungsgleich sind, so daß die Öffnungen bei gestapelten Karten rohrförmige Kanäle bilden, der umlaufende Rand der Gewebekarten sowie eine ringförmige Fläche des Gewebes, die einige der Öffnungen einschließt, mit Dichtmittel gefüllt sind, wobei sich die Öffnungen mit und ohne Dichtmittel einer Reihe einer Gewebekarte und bei den rohrförmigen Kanälen abwechseln.

Die Erhitzungstemperatur liegt zwischen + 65 °C und 80 °C.

Damit die zugeführte Flüssigkeit innerhalb einer möglichst kurzen Zeit auf die jeweilige Erhitzungstemperatur aufgeheizt werden und anschließend in der gleichen Zeit wieder abgekühlt werden kann, ist die Apparatur, in der erhitzt wird, zweckmäßig mit je einem Anschluß für Heiz- und Kühlmedium ausgestattet.

Die konstruktive Gestaltung der Wärmeübertrager sollte derart sein, daß die Differenz zwischen der Erhitzungstemperatur für die Lösung und der Temperatur des Heizmediums möglichst klein ist, was wegen der daraus resultierenden geringen Wandtemperatur größtmögliche Produktschonung zur Folge hat.

Die Aufheiz- und Abkühlzeit soll je 2 Sekunden betragen. Die Haltezeit liegt zwischen 0,5 und 5 Sekunden.

Die Protein-Präparation kann beispielsweise ein Gewebe-Thromboplastin enthaltendes Therapeutikum oder ein Gewebe-Thromboplastin enthaltendes Diagnostiktum, beispielsweise ein Quick-Reagenz sein.

Im folgenden Beispiel wird die Virusinaktivierung durch Kurzzeiterhitzung ohne Einschränkung der Erfindung am Beispiel von ^{R}Thromborel S, einem gewebethromboplastinhaltigen Quick-Reagenz aus Human-Plazenta der Behringwerke AG gezeigt. Der Erfolg der Inaktivierung wurde geprüft. Bei einer Erhitzungstemperatur von über 45°C, vorzugsweise von mindestens 65°C wurde eine vollständige Virusinaktivierung gefunden. Die diagnostischen Eigenschaften des kurzzeiterhitzten Reagenzes wurden mit denen von unbehandeltem ^{R}Thromborel S verglichen. Mindestens bis zu einer Erhitzungstemperatur von 75°C wurden keine nachteiligen Veränderungen dieser Eigenschaften beobachtet.

### Beispiel

### Durchführung der Kurzzeiterhitzung

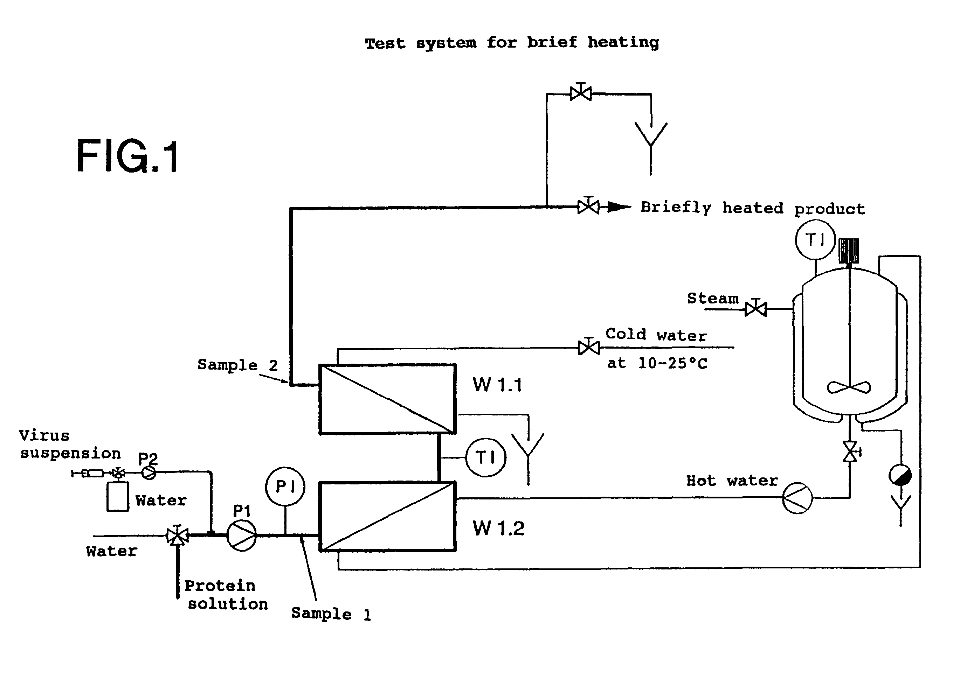

Dazu wurden zwei Wärmeübertrager (W 1.1 und W 1.2, Abb. 1) in eine gemeinsame Einspannvorrichtung eingebaut, die im wesentlichen handelsüblichen Ultrafiltrations-Kassettenhaltern entspricht. Die modular aufgebauten Wärmeübertrager waren durch eine speziell konstruierte Zwischenplatte voneinander getrennt.

Dadurch war es möglich, die Wärmeübertrager so zu schalten, daß die zugeführte Flüssigkeit innerhalb von 2 Sekunden auf die jeweilige Erhitzungstemperatur aufgeheizt wurde und anschließend in der gleichen Zeit wieder abkühlte. Zu diesem Zweck war die Apparatur mit je einem Anschluß für Heiz- und Kühlmedium ausgestattet. Die Haltezeit bei der eingestellten Erhitzungstemperatur betrug etwa 1,5 Sekunden.

Vor Verfahrensbeginn wurde das gesamte System produktseitig mit Wasser gespült und auf Arbeitstemperatur aufgeheizt. Hierzu förderte die Pumpe P1 (Abb. 1) so lange Heizmedium durch die Anlage, bis die gewünschte Erhitzungstemperatur erreicht war. Nun wurde von Wasser auf die zu erhitzende Lösung umgestellt und in Kontrollversuchen anschließend die Pumpe P2 zur Dosierung der Virussuspension eingeschaltet. Proben wurden vor Erhitzen und nach der experimentell ermittelten Mindestversuchszeit von 20 bzw. 48 Sekunden gezogen.

### Ergebnis der Virusinaktivierung

Zur Kontrolle wurde eine Lösung von Gewebe-Thromboplastin bei Raumtemperatur durch die Anlage geleitet. Die Ergebnisse zeigen, daß für das hüllfreie und hitzebeständige Poliovirus bei allen getesteten Erhitzungstemperaturen (65-75°C) eine vollständige Virusinaktivierung von mehr als 5 Zehnerpotenzen erreicht wurde. Ebenfalls vollständig war die Inaktivierung im Falle des Herpesvirus HSV-1.

Die Inaktivierung wurde für 2 verschiedene Ausgangstiter geprüft (ca. 3,5 und >5,0) und war in beiden Fällen vollständig. Im Kontrollexperiment war dagegen keine oder nur eine geringe Virusinaktivierung nachweisbar.

### Vergleich von kurzzeiterhitztem mit unbehandeltem Gewebe-Thromboplastin

^{R}Thromborel S wurde ohne Zudosierung von Virussuspension in der obigen Anlage bei verschiedenen Temperaturen kurzzeiterhitzt. Als Kontrolle diente unbehandeltes Material. Alle Präparationen wurden gefriergetrocknet und vor der Testung im gleichen Volumen dest. Wasser rekonstitutiert. Die nachfolgend dargestellten Ergebnisse wurden mit einer repräsentativen Charge erhalten.

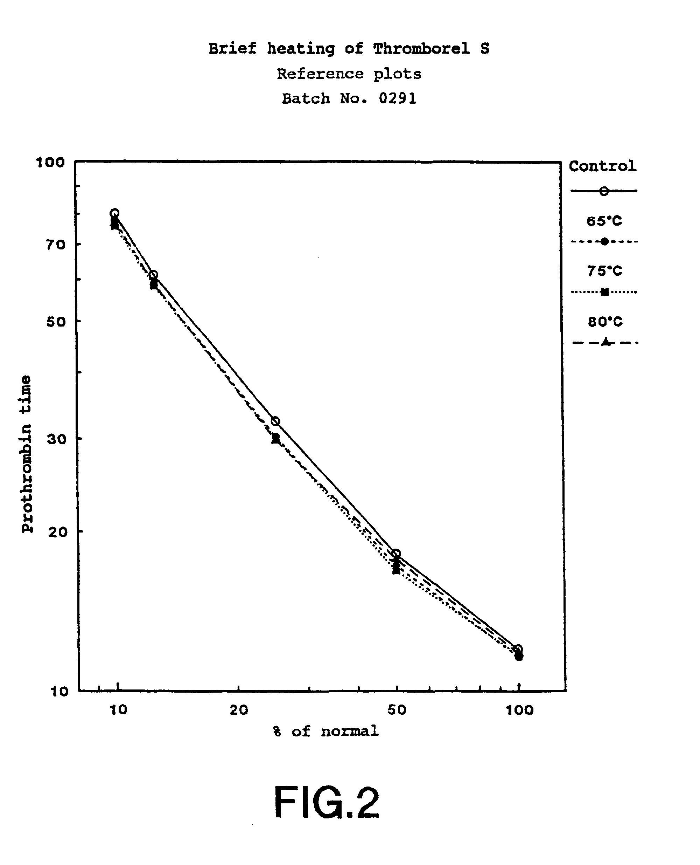

Zur Erstellung der Bezugskurven (Abb. 2) wurde Standard-Human-Plasma der Behringwerke AG unverdünnt und mit isotonischer Kochsalzlösung verdünnt als Probe eingesetzt. Probe (100 µl) und Reagenz (200 µl) wurden gemischt und die Gerinnungszeit am Koagulometer nach Schnitger & Gross gemessen. Die Bezugskurven von erhitztem und unbehandelten ^{R}Thromborel S sind im wesentlichen identisch.

Die Empfindlichkeit von Quick-Reagenzien wird durch den ISI (international sensitivity index) ausgedrückt. Die ISI-Werte von erhitztem und unbehandeltem ^{R}Thromborel S, bestimmt an Plasmen gesunder und oral antikoagulierter Probanden im Vergleich mit einem Referenz-Thromboplastin, sind ununterscheidbar.

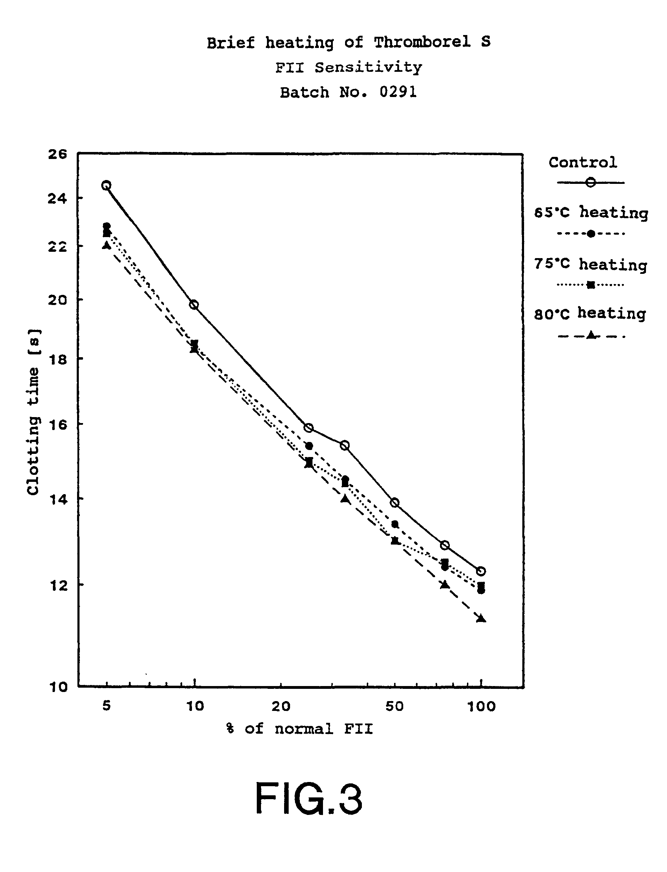

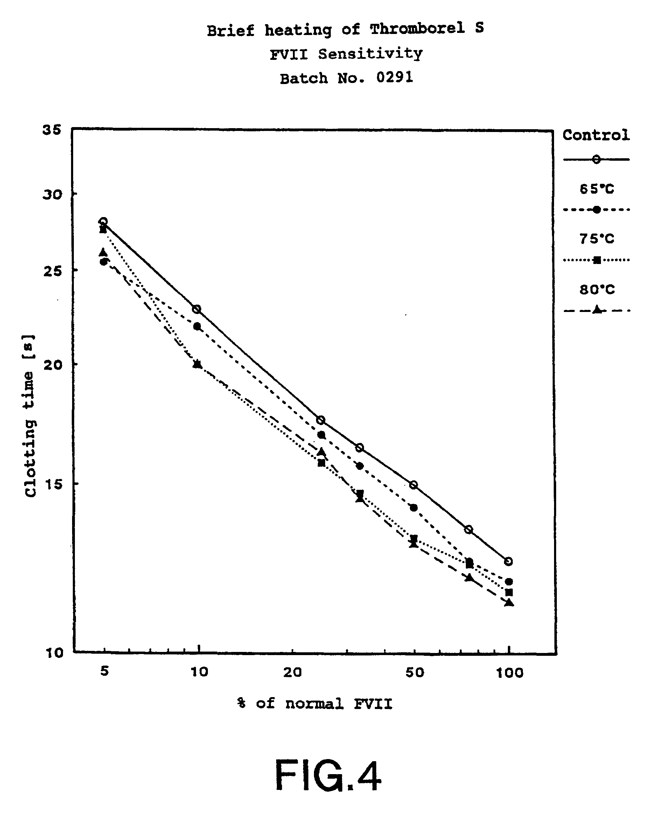

Zur Charakterisierung der Empfindlichkeit von erhitztem und unbehandeltem ^{R}Thromborel S auf die Gerinnungsfaktoren II, V, VII und X wurde Standard-Human-Plasma mit dem entsprechenden Gerinnungsfaktoren-Mangelplasma der Behringwerke AG gemischt, so daß sich Aktivitäten des betreffenden Faktors zwischen 5 und 100 % der Norm einstellten. Die Gerinnungszeiten der Mischungen (100 µl) wurden nach Zugaben von 200 µl von erhitztem oder unbehandeltem ^{R}Thromborel S am Koagulometer nach Schnitger & Gross bestimmt. In Abb. 3 und 4 ist die Gerinnungszeit gegen den Gehalt an Faktor II bzw. F VII in Prozent der Norm aufgetragen; hier ergab sich eine im Vergleich zur Kontrolle gleich gute Empfindlichkeit auf die geprüften Faktoren. Die (nicht gezeigten) Empfindlichkeiten für Faktor V und Faktor X waren im Rahmen der Meßgenauigkeit ununterscheidbar.

## Patentansprüche

1. Verfahren zur Inaktivierung von Viren in Präparationen eines Proteins aus der Gruppe der Plazentenproteine ohne Beeinträchtigung der Eigenschaften dieses Proteins, **dadurch gekennzeichnet, daß** eine Lösung dieser Präparation in einem Wärmeübertrager mit indirekter Beheizung bei einer Aufheiz- und Abkühlzeit von 2 Sekunden, einer Haltezeit zwischen 0.5 und 5 Sekunden und einer Erhitzungstemperatur zwischen +65 °C und 80 ° C erhitzt wird.

2. Verfahren nach Anspruch 1, **dadurch gekennzeichnet, daß** eine Lösung von Gewebe-Thromboplastin erhitzt wird.

## Claims

1. A process for the inactivation of viruses in preparations of a protein from the group of placental proteins without impairing the properties of this protein, which comprises heating a solution of this preparation in a heat exchanger with indirect heating, with a heating and cooling time of 2 seconds, with a dwell time between 0.5 and 5 seconds and at a heating temperature between +65° and 80°C.

2. The process as claimed in claim 1, wherein a solution of tissue thromboplastin is heated.

## Revendications

1. Procédé pour l'inactivation de virus dans des préparations d'une protéine appartenant au groupe des protéines placentaires, sans altération des propriétés de cette protéine, **caractérisé en ce que** l'on chauffe une solution de cette préparation, dans un dispositif de transfert thermique à chauffage indirect, pendant un temps de chauffage et de refroidissement de 2 secondes, avec un temps de séjour compris entre 0,5 et 5 secondes et une température de chauffage comprise entre +65 et +80°C.

2. Procédé selon la revendication 1, **caractérisé en ce que** l'on chauffe une solution de thromboplastine tissulaire.
